(19) Europäisches Patentamt
European Patent Office
Office européen des brevets

(11) Publication number: **0 423 926 B1**

(12) **EUROPEAN PATENT SPECIFICATION**

(45) Date of publication of patent specification: **03.08.94**

(51) Int. Cl.[5]: **C07C 255/50**, C09K 19/30

(21) Application number: **90309177.5**

(22) Date of filing: **21.08.90**

The file contains technical information submitted after the application was filed and not included in this specification

(54) **Difluorobenzonitrile derivatives.**

(30) Priority: **16.10.89 JP 268675/89**

(43) Date of publication of application: **24.04.91 Bulletin 91/17**

(45) Publication of the grant of the patent: **03.08.94 Bulletin 94/31**

(84) Designated Contracting States: **CH DE FR GB LI**

(56) References cited:
**EP-A- 0 205 998**
**EP-A- 0 316 715**

**PATENT ABSTRACTS OF JAPAN, vol. 11, no. 317 (C-452)(2764), 15 October 1987; & JP-A-62 103 057**

(73) Proprietor: **Chisso Corporation**
**6-32, Nakanoshima 3-chome**
**Kita-ku**
**Osaka-shi Osaka-fu(JP)**

(72) Inventor: **Kitano, Kisei**
**3-3-105, Tendai 4-chome**
**Chibashi, Chibaken(JP)**
Inventor: **Goto, Yasuyuki**
**462-2, Saehiro,**
**Ichiharashi**
**Chibaken(JP)**
Inventor: **Ogawa, Tetsuya**
**882, Chigusashinden**
**Futtsushi, Chibaken(JP)**

(74) Representative: **Lamb, John Baxter et al**
**MARKS & CLERK,**
**57-60 Lincoln's Inn Fields**
**London WC2A 3LS (GB)**

EP 0 423 926 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a difluorobenzonitrile derivative used as a component of liquid crystal materials and a liquid crystal composition containing the same.

Display devices having liquid crystals applied thereto utilize electrooptical effects based upon the anisotropies of dielectric constant and electric conductivity of liquid crystal substances.

Liquid crystal display modes include various ones such as dynamic scattering mode, twisted nematic mode, supertwisted nematic mode, phase transition mode, DAP mode, guest-host mode, etc. Properties required for liquid crystal substances used for liquid crystal display vary depending upon the respective liquid crystal display modes, but a broad mesomorphic range, stability to moisture, air, light, heat, electricity, etc. and the like are properties required in common to any display modes. Further, when liquid crystal substances are used for liquid crystal display devices, it is also desired that the response speed of display elements is high and the devices can be driven at a voltage as low as possible. However, there is no single compound which alone satisfies all of these requirements, but practically, mixtures of several kinds of liquid crystal compounds or liquid crystalline mixtures obtained by further mixing compounds similar to the above liquid crystals have been used.

In recent years, with broadening of the application ranges of liquid crystal display elements, characteristics required for liquid crystal materials have been becoming severer. Particularly, in the case of TN mode cell, it is necessary, as reported in G. Bauer, Mol. Cryst. Liq. Cryst. 63, 43 (1981), to set the product of the optical anisotropy value ($\Delta n$) of liquid crystal materials filled in the cell by the cell thickness d ($\mu m$) to a definite value, in order to prevent the occurrence of the interference fringes becoming a cause of damaging the appearance of display cells. In the case of practically used liquid crystal cells, the value of $\Delta n \times d$ has been set to any one of 0.5, 1.0, 1.6 or 2.2. Further, in the case where the value of $\Delta n \times d$ is a specified one, if the value of $\Delta n$ is large, it is possible to reduce the value d, whereby the response characteristic is improved. Further, if the d value is small, the intensity of the electric field increases under the same impressed voltage, whereby a low voltage drive is possible. Further, if the value of $\Delta n \times d$ is large, the contrast of display cell is improved. Thus a compound having a large $\Delta n$ value has been required.

Further, the dielectric anisotropy value $\Delta\epsilon$ affects the threshold voltage value of liquid crystal cells. Namely, the larger the $|\Delta\epsilon|$ value, the lower the threshold voltage value.

SUMMARY OF THE INVENTION.

The object of the present invention is to provide a compound satisfying the above-mentioned requirements and capable of improving various characteristics of display elements when it is used as a component of liquid crystal compositions and a liquid crystal composition containing the compound.

The present invention resides in a difluorobenzonitrile derivative expressed by the formula

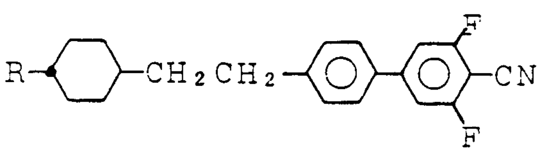
(I)

wherein R represents an alkyl group of 1 to 10 carbon atoms, and a liquid crystal composition comprising at least two components at least one of which is said difluorobenzonitrile derivative.

DETAILED DESCRIPTION OF PREFERRED EMBODIMENTS

In the compound of the formula (I), R is preferred to be an alkyl group of 2 to 6 carbon atoms.

The compound provided by the present invention is a liquid crystal compound having a positive large $\Delta\epsilon$ value and also a large $\Delta n$ value. Further, the compound of the present invention has a liquid crystal phase within a temperature range preferred for liquid crystal display devices and is stable to moisture, air, light, heat, electricity, etc. and further is usable as a component suitable for liquid crystal compositions constituting liquid crystal display devices.

As difluorobenzonitrile derivatives similar to the compound of the present invention, compounds expressed by the following formulas (a), (b) and (c) are disclosed in Japanese patent application laid-open No. Sho 58-167528/1983 (the corresponding U.S.P. No. 4,551,264; EP-316,715), Japanese patent application laid-open No. Hei 1-131144/1989 (the corresponding U.S.P. 4,853,152; EP-316,715) and Japanese patent application laid-open No. Sho 62-103057:

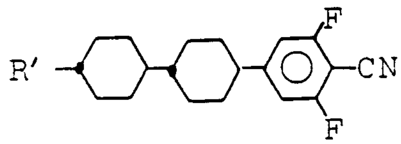

(a)

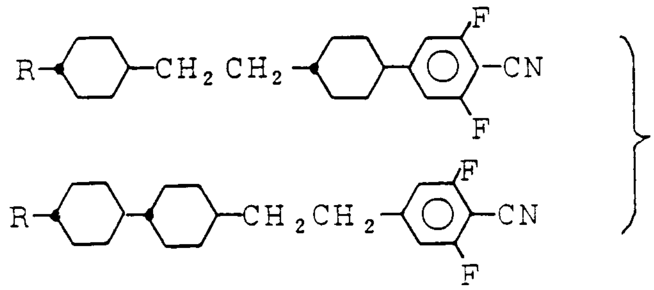

(b)

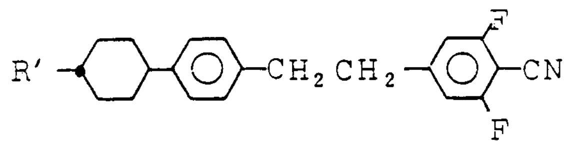

(c)

wherein R′ represents a linear alkyl group.

These compounds have a Δn value as small as about 0.10, whereas the compound of the present invention is superior in that it has a Δn value as large as about 0.18, close to about twice the above value.

Preparation of the compound

The compound (I) of the present invention may be prepared for example according to the following equations:

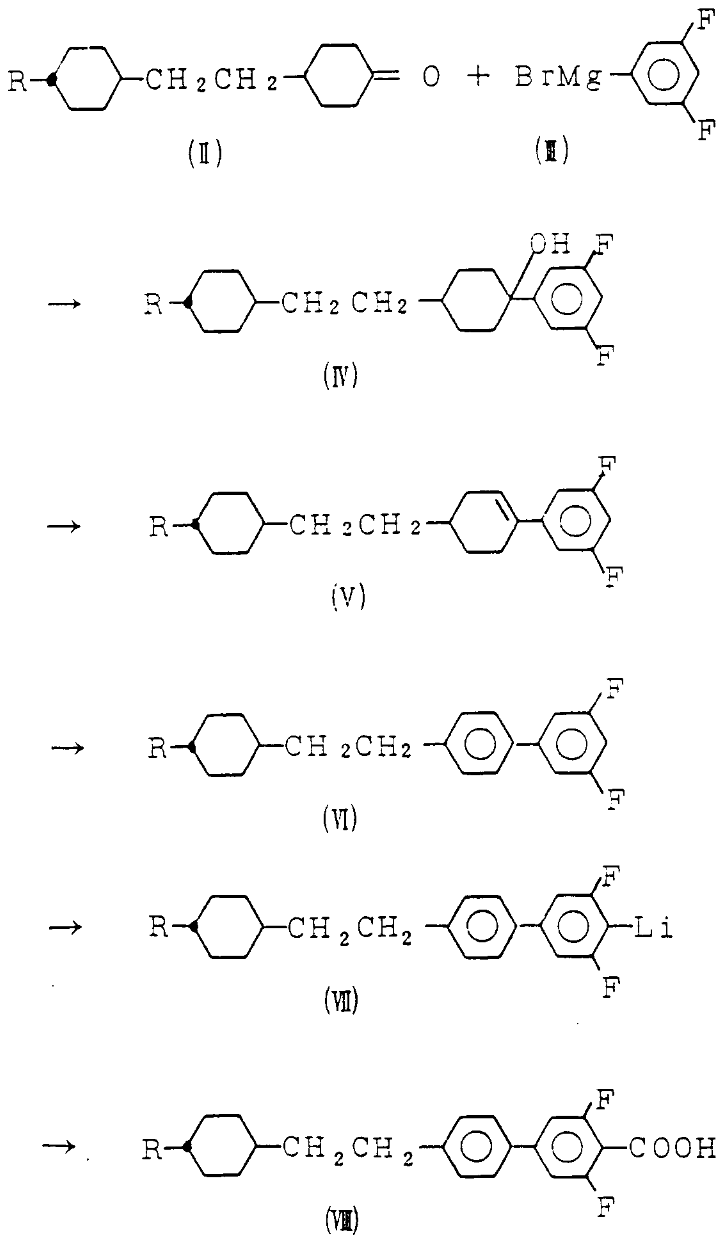

(II) (III)

(IV)

(V)

(VI)

(VII)

(VIII)

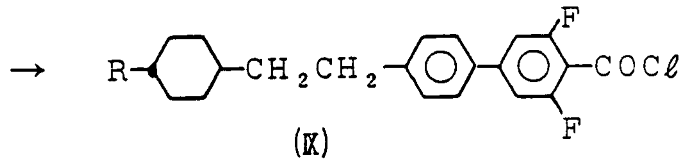

(IX)

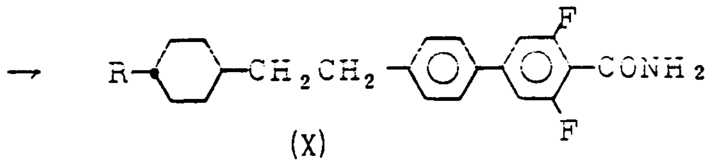

(X)

→ (I)

Namely, a ketone derivative expressed by the formula (II) is subjected to a coupling reaction with a Grignard reagent obtained from 1-bromo-3,5-difluorobenzene to obtain an alcohol derivative (IV), which is subjected to dehydration reaction in the presence of an acid catalyst such as an acid e.g. sulfuric acid, hydrochloric acid, etc., a Lewis acid e.g. anhydrous aluminum chloride, ferric chloride, $TiC\ell_4$, etc., or an organic acid e.g. benzenesulfonic acid, p-toluenesulfonic acid, etc. to obtain a cyclohexene derivative (V), which is reacted with an oxidation-dehydrogenation agent such as chloranil, DDQ, etc. in the presence of an inert solvent such as toluene, xylene, etc. to obtain a difluorobiphenyl derivative (VI), which is lithiated by a lithiating agent such as alkyllithiums, etc. to obtain a compound (VII), which is reacted with $CO_2$ gas to obtain a carboxylic acid compound (VIII), which is reacted with a chlorinating agent such as thionyl chloride, etc. to obtain an acid chloride (IX), which is reacted with ammonia to obtain an acid amide derivative (X), which is reacted with a dehydrating agent such as a salt of p-toluenesulfonic acid, etc. to obtain the objective compound (I).

Since the compound of the present invention has a superior compatibility with other liquid crystalline compounds, admixture of the compound with the above compounds or mixtures thereof makes it possible to prepare liquid crystal materials suitable to various use applications.

Liquid crystal components used together with the compound of the formula (I) as component(s) of the liquid crystal composition of the present invention have no particular limitation, but liquid crystal compounds expressed by the following formulas (i) to (xxxiii) may be exemplified:

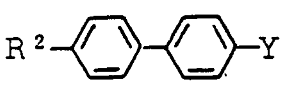

(i)

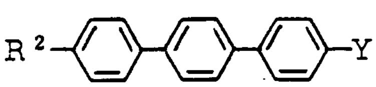

(ii)

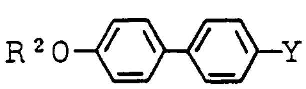

(iii)

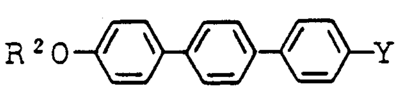

(iv)

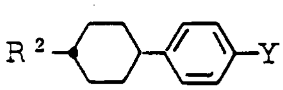

(v)

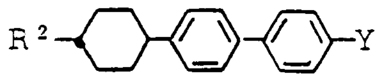 (vi)

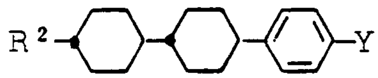 (vii)

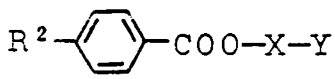 (viii)

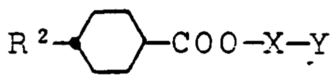 (ix)

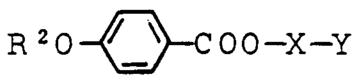 (x)

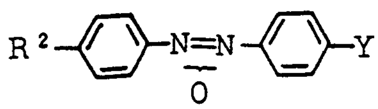 (xi)

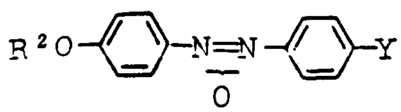 (xii)

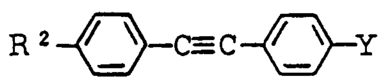 (xiii)

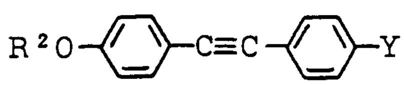 (xiv)

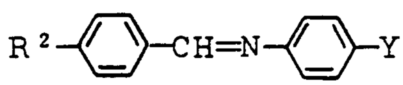 (xv)

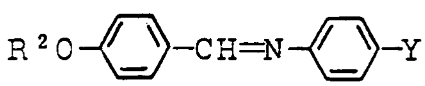 (xvi)

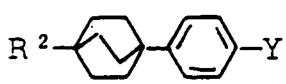 (xvii)

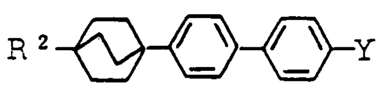 (xviii)

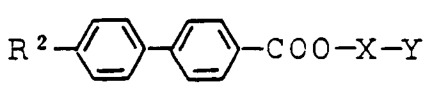 (xix)

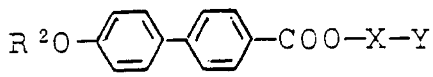
(xx)

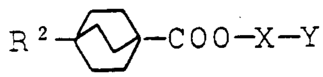
(xxi)

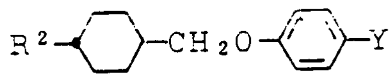
(xxii)

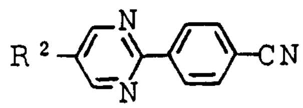
(xxiii)

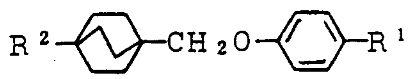
(xxiv)

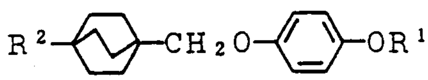
(xxv)

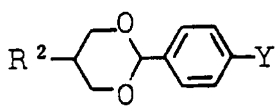
(xxvi)

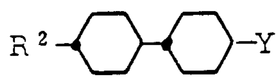
(xxvii)

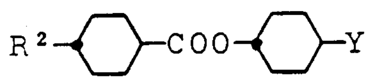
(xxviii)

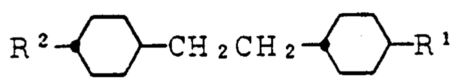
(xxix)

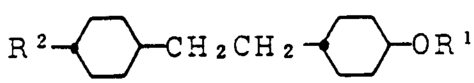
(xxx)

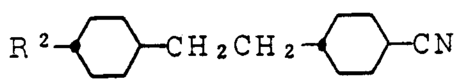
(xxxi)

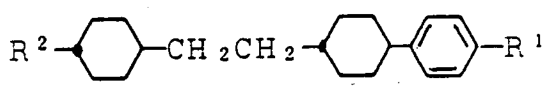
(xxxii)

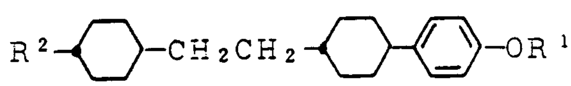
(xxxiii)

In the formulas (i) to (xxxiii), X represents

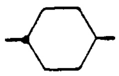 , 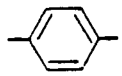 , 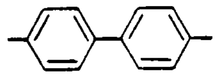 or 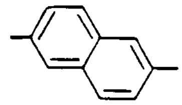 ,

Y represents -CN, a halogen atom, $R^1$ or $OR^1$, $R^2$ and $R^1$ each represent an alkyl group and the hydrogen atom(s) of these

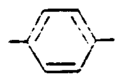

may be replaced by halogen atom(s).

By using the compound of the present invention, it is possible to notably broaden the chosen range of liquid crystalline compounds when several kinds of liquid crystalline compounds are mixed, to prepare liquid crystal materials suitable to various display devices.

(Example)

The present invention will be described in more detail by way of Examples, but it should not be construed to be limited thereto.

Example 1

Preparation of 4-[4-(trans-4-butylcyclohexylethyl)phenyl]-2,6-difluorobenzonitrile

(i) A solution of 1-bromo-3,5-difluorobenzene (100 g, 0.52 mol) dissolved in anhydrous tetrahydrofuran (300 mℓ) was slowly dropwise added to shaved magnesium (12.6 g, 0.52 mol) in nitrogen current with stirring to obtain a tetrahydrofuran solution of 3,5-difluorophenylmagnesium bromide, followed by dropwise adding to the solution, a solution of 4-(trans-4-butylcyclohexylethyl)cyclohexane (137 g, 0.52 mol) dissolved in anhydrous tetrahydrofuran (100 mℓ) over one hour while keeping the reaction temperature at 5° to 10°C, thereafter raising the temperature of the reaction solution up to 35°C to age it for one hour, adding 3N hydrochloric acid (200 mℓ), extracting the reaction product with toluene (300 mℓ), washing the extract solution with water till the washing water became neutral to obtain a toluene solution of 3,5-difluoro-1-[1-hydroxy-4-(trans-4-butylcyclohexylethyl)cyclohexyl]benzene, adding to the solution, p-toluenesulfonic acid hydrate (4.5 g, 0.02 mol), removing water formed under reflux on heating by azeotropy with toluene, washing the toluene solution after completion of the reaction, with water till the washing water became neutral, distilling off toluene and recrystallizing the residue from a mixed solvent of ethanol and heptane (1:1) to obtain 3,5-difluoro-1-[4-(trans-4-butylcyclohexylethyl)cyclohexen-1-yl]benzene (65 g, 0.18 mol). This compound exhibited liquid crystal phases and the crystal-nematic phase transition point (C-N point) was 49°C and the nematic phase-isotropic liquid phase transition point (N-I point) was 66°C.

(ii) 3,5-Difluoro-1-[4-(trans-4-butylcyclohexylethyl)cyclohexen-1-yl]benzene (50 g, 0.16 mol) obtained above in (i) and chloranil (116 g, 0.47 mol) were added to xylene (250 mℓ), followed by heating the mixed solution under reflux for 20 hours, cooling the resulting solution down to room temperature, filtering off a deposited hydroquinone derivative, washing the filtrate with a dilute alkali aqueous solution, further washing with water till the washing water became neutral, drying over anhydrous sodium sulfate, distilling off xylene from the organic layer, dissolving the residue in heptane, purifying the solution by silica gel chromatography and recrystallizing from ethanol to obtain 4′-(trans-4-butylcyclohexylethyl)-3,5-difluorobiphenyl (16 g, 0.045 mol). This compound exhibited a crystal-isotropic liquid phase transition point (C-I point) of 56°C and an N-I point of 42°C and was a monotropic liquid crystal.

(iii) 4′-(Trans-4-butylcyclohexylethyl)-3,5-difluorobiphenyl (15 g, 0.042 mol) obtained above in (ii) was dissolved in tetrahydrofuran (100 mℓ), followed by cooling the solution down to -80°C, adding to the solution, a hexane solution (40 mℓ) of butyllithium at -80°C over 17 minutes, further agitating the mixture at this temperature for one hour, blowing $CO_2$ gas therein at -50°C, returning the temperature to room

temperature over one hour, adding 1N hydrochloric acid (100 mℓ), extracting a freed carboxylic acid with a mixed solvent (200 mℓ) of toluene and ether (1:1), washing the resulting organic layer with water till the washing water became neutral, drying over anhydrous magnesium sulfate, distilling off the solvent and recrystallizing the remaining solids from a mixed solvent of toluene and methanol (10:1) to obtain 4-[4-(trans-4-butylcyclohexylethyl)phenyl]-2,6-difluorobenzoic acid (12 g, 0.029 mol).

(iv) 4-[4-(Trans-4-butylcyclohexylethyl)phenyl]-2,6-difluorobenzoic acid (11 g, 0.027 mol) obtained above in (iii) was reacted with thionyl chloride (20 mℓ) under reflux on heating for 5 hours, followed by distilling off thinyl chloride under reduced pressure, dissolving the residue in toluene, adding the solution to a mixture of ice (50 g) and aqueous ammonia (50 mℓ), agitating the mixture, filtering off deposited solids, drying the solids under reduced pressure, adding to the solids, toluene (50 mℓ), p-toluenesulfonic acid chloride (11 g, 0.057 mol) and pyridine (9 mℓ), heating the mixture under reflux for 20 hours, allowing the reaction solution to cool down, adding water (100 mℓ), agitating the mixture, washing the toluene solution with 2N NaOH aqueous solution (100 mℓ), further washing with water till the washing water became neutral, drying the toluene layer over anhydrous magnesium sulfate, filtering off the drying agent, distilling off toluene, dissolving the residue in heptane, purifying according to alumina chromatography and recrystallizing from ethanol to obtain the objective 4-[4-(trans-4-butylcyclohexylethyl)phenyl]-2,6-difluorobenzonitrile (6.3 g, 0.017 mol). This compound exhibited a C-I point of 71 °C and an N-I point of 108 °C.

The following compounds are obtained in the same manner:

4-[4-(trans-4-methylcyclohexylethyl)phenyl]-2,6-difluorobenzonitrile
4-[4-(trans-4-ethylcyclohexylethyl)phenyl]-2,6-difluorobenzonitrile
4-[4-(trans-4-propylcyclohexylethyl)phenyl]-2,6-difluorobenzonitrile
4-[4-(trans-4-pentylcyclohexylethyl)phenyl]-2,6-difluorobenzonitrile
4-[4-(trans-4-hexylcyclohexylethyl)phenyl]-2,6-difluorobenzonitrile
4-[4-(trans-4-heptylcyclohexylethyl)phenyl]-2,6-difluorobenzonitrile
4-[4-(trans-4-octylcyclohexylethyl)phenyl]-2,6-difluorobenzonitrile
4-[4-(trans-4-nonylcyclohexylethyl)phenyl]-2,6-difluorobenzonitrile
4-[4-(trans-4-decylcyclohexylethyl)phenyl]-2,6-difluorobenzonitrile

Example 2 (Composition)

A liquid crystal composition A consisting of trans-4-propylcyclohexylbenzonitrile 30 wt. parts, trans-4-pentylcyclohexylbenzonitrile 40 wt. parts and 4-trans-4-heptylcyclohexylbenzonitrile 30 wt. parts exhibits an N-I point of 52.1 °C, a $\Delta\epsilon$ at 25 °C of 10.7, a $\Delta n$ of 0.119, and a viscosity at 20 °C ($\eta_{20}$) of 22.3 cp.

This composition was sealed in a TN mode cell of 9 µm thick and the threshold voltage was measured to give 1.60 V.

When a compound of the present invention, 4-[4-trans-4-butylcyclohexylethyl)phenyl]-2,6-difluorobenzonitrile (15 parts by weight) obtained in Example 1 was added to the above liquid crystal composition A (85 parts by weight), the resulting liquid crystal mixture exhibited an N-I point elevated up to 56.7, a $\Delta\epsilon$ notably increased up to 12.5, a $\Delta n$ notably increased up to 0.128 and a $\eta_{20}$ elevated up to 30.1 cp. When the mixture was sealed in the above TN mode cell, the threshold voltage decreased down to 1.42 V.

Comparative examples

The fore-mentioned compounds (a), (b) and (c) each having a similar substituent on the terminal phenyl ring to that of the compound (I) of the present invention were compared with the compound (I) as regards $\Delta n$.

The respective $\Delta n$ values at 25 °C of the liquid crystal mixtures each obtained by adding the compound (a), (b) or (c) (15 parts by weight) to the liquid crystal composition A (85 parts by weight) are shown in Table 1 together with the $\Delta n$ value in Example 2.

Table 1

| No. | Compound added in 15 wt.% | Δn |
|---|---|---|
| | Liquid crystal composition A | 0.119 |
| Compar. ex. 1 | 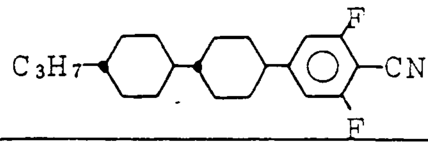 (a) | 0.119 |
| Compar. ex. 2 | 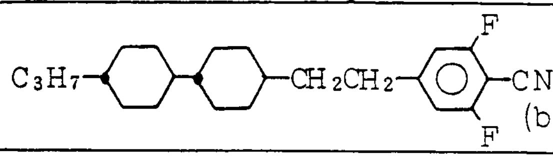 (b) | 0.119 |
| Compar. ex. 3 | 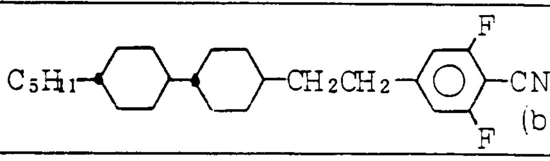 (b) | 0.118 |
| Compar. ex. 4 | 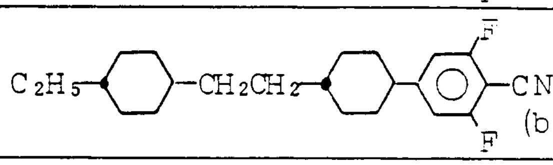 (b) | 0.114 |
| Compar. ex. 5 | 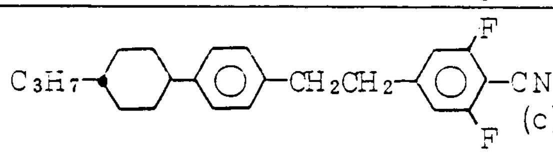 (c) | 0.116 |
| Example 2 | 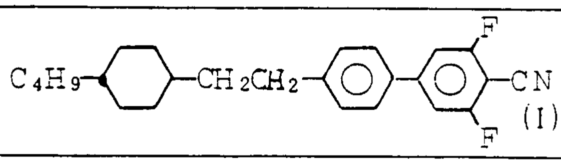 (I) | 0.128 |

As apparent from Table 1, the Δn value of the compound (I) of the present invention is far larger than those of compounds (a), (b) and (c).

## Claims

**1.** Difluorobenzonitrile derivatives of the formula

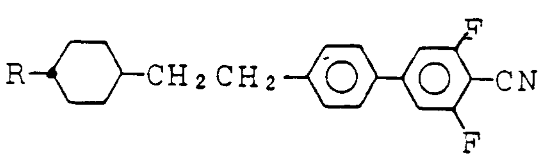

(I)

wherein R represents an alkyl group of 1 to 10 atoms.

2. Difluorobenzonitrile derivatives according to claim 1 wherein R represents an alkyl group of 2 to 6 carbon atoms.

3. A liquid crystal composition comprising at least two components, at least one of which is a difluorobenzonitrile derivative as set forth in claim 1.

### Patentansprüche

1. Difluorobenzonitrilderivate der Formel

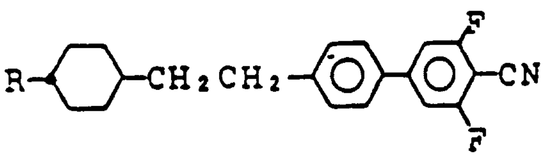

(I)

worin R eine Alkyl-Gruppe mit 1 bis 10 Kohlenstoffatomen bedeutet.

2. Difluorobenzonitrilderivate nach Anspruch 1, worin R eine Alkyl-Gruppe mit 2 bis 6 Kohlenstoffatomen bedeutet.

3. Flüssigkristall-Zusammensetzung aus mindestens zwei Bestandteilen, wovon mindestens eine ein Difluorobenzonitrilderivat gemäß Anspruch 1 ist.

### Revendications

1. Dérivés de difluorobenzonitrile de la formule:

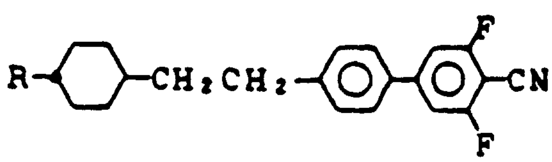

(I)

dans laquelle R représente un groupe alkyle de 1 à 10 atomes de carbone.

2. Dérivés de difluorobenzonitrile selon la revendication 1, dans lesquels R représente un groupe alkyle de 2 à 6 atomes de carbone.

3. Composition liquide cristalline comprenant au moins deux composants dont au moins un est un dérivé de difluorobenzonitrile tel qu'exposé dans la revendication 1.